# EUROPEAN PATENT APPLICATION

(11) **EP 2 138 172 A1**
(43) Date of publication of application: **30.12.2009**
(21) Application number: 08738680.1
(22) Date of filing: 19.03.2008
(51) Int. Cl.: A61K 31/11, A61K 31/352, A61K 31/4406, A61K 36/00, A61P 31/22

(54) **ANTI-VIRAL AGENT**

(30) Priority: 19.03.2007 JP 2007071409
(71) Applicant: Tsuchida, Yuuzou, Tokyo 1420062 (JP)
(72) Inventor: WATANABE, Kunitomo, Gifu-shi Gifu 502-0816 (JP); KOKETSU, Mamoru, Gifu-shi Gifu 502-0813 (JP); MURAYAMA, Tsugiya, Kahoku-gun Ishikawa 920-0274 (JP); TSUCHIDA, Kotarou, Tokyo 142-0062 (JP); SAKURAI, Daisuke, Tokyo 142-0062 (JP); KAWABE, Mitsuo, Kawaguchi-shi Saitama 334-0056 (JP)
(74) Representative: Müller-Boré & Partner Patentanwälte
(86) International application number: PCT/JP2008/055087
(87) International publication number: WO 2008/123102

(57) **Abstract**

Provided is an antiviral agent with high antiviral activities and low side effects (cytotoxicity). Specifically provided is an antiviral agent comprising as an effective component at least one member selected from the group consisting of 5,7,4'-trihydroxy-3',5'-dimethoxyflavone, 3-hydroxypyridine, p-hydroxybenzaldehyde and vanillin.

## Description

### TECHNICAL FIELD

The present invention relates to an antiviral agent.

### BACKGROUND ART

A virus is an extremely small pathogen which is incapable of self-propagating and parasitically reproduces in animals, plants and bacteria. Viruses are divided into animal viruses, plant viruses and microbe viruses.
Human cytomegalovirus exhibits high species-specificity and does not proliferate in cells other than cells with a human origin. To propagate the virus, primary culture cells derived from human fetal skin, muscle, preputium, lung or the like, and established cell lines such as MRC-5 and WI-38 are used. In infected cells, intranuclear inclusion bodies (basic) and intracytoplasmic inclusion bodies are observed. Transplacental infection (transovarial transmission) can occur, which causes a stillbirth or premature birth and congenital infectious disease (CID). Acquired infections can also occur. In that case, generally after inapparent infections, a virus in the latent stage becomes activated upon immunosupressive therapy and/or use of steroid hormones in a susceptible host including an AIDS patient and/or cancer patient, which causes serious opportunistic infectious diseases such as pneumonia, retinitis or colitis.
A variety of antiviral agents for preventing and treating these diseases caused by the virus have been developed. For instance, Gancicrovir (GCV), Cidofovir (CDV), Foscarnet (PFA), Fomivirsen and the like are known. Among these, GCV has been widely used but recently a GCV-resistant virus is becoming a problem. In addition, there is also a problem in that the drug has strong side effects (cell cytotoxicity). An agent with no side effects and with effective antiviral activities even against drug-resistant viruses has been wanted.

Influenza virus (influenza virus, flu virus) is a virus which infects human and causes an infectious disease, influenza. The virus is categorized into type A, type B and type C.
In addition, some influenza viruses infect domestic fowls such as poultry and cause highly pathogenic avian influenza (fowl plague) which is a fatal infectious disease and legally designated infectious disease, thereby causing huge damages to the poultry industry. As of 2006, three types of anti-influenza drugs, namely amantadine, zanamivir, oseltamivir (trade name Tamiflu) are available. Meanwhile, it has been already reported that viruses acquired resistance against these drugs, e.g. amantadine-resistant influenza viruses, zanamivir (oseltamivir)-resistant influenza viruses, and viruses resistant to both zanamivir and oseltamivir emerged.
Accordingly, a drug with no side effects and having high antiviral activities even against drug resistant viruses has been wanted.

In the meantime, it is known for a long time that a bamboo (Sasa) extract has antimicrobial activities. For example, it has been reported that the extract has an antimicrobial effect against *Staphylococcus aureus, Pseudomonas aeruginosa* and *Escherichia coli*, which are the causative bacteria of wound infection (Patent Documents 1 and 2), as well as against *Helicobacter pylori* which is thought to be the causative bacteria of gastric ulcer. After World War II, an animal experiment revealed that Kumazasa had suppressive activities against liver cancer in a rat and several pharmacological studies (Non-patent Document 1) have been carried out from an aspect of anti-cancer activities. Further, studies on an excellent preservative activity (Non-patent Document 2) and antimicrobial activity (Non-patent Document 3) which Kumazasa has have also been carried out. However, in most of these studies, nothing more than the analysis of organic acids by using gas chromatography has been reported. There are few reports which disclose the isolation and purification of the essential components which provide an antimicrobial effect.
It has also been known that coumaric acid and derivatives thereof have an antimicrobial activity against *Escherichia coli, Staphylococcus aureus* and *Pseudomonas aeruginosa* (Patent Document 3).
Moreover, it has also been known that Kumazasa extract contains phenylpropanoids such as coumaric acid, ferulic acid, coffeic acid and vanillin, 3-hydroxypyridine and the like, and a mixture of these shows an antimicrobial activity against *Escherichia coli, Staphylococcus aureus* and *Pseudomonas aeruginosa* (Patent Document 4).
Nevertheless, details of each component in Kumazasa extract and antiviral activities thereof have not known.

Patent Document 1: WO00/067707
Patent Document 2: Japanese Laid-open Patent Application (Kokai) No. 2003-201247
Patent Document 3: Japanese Laid-open Patent Application (Kokai) No.2004-359626
Patent Document 4: Japanese Laid-open Patent Application (Kokai) No.2006-36731
Non-patent Document 1: M. Shibata, K. Kubo, M. Onoda, Folia Pharmacol. Jpn, 72, 531-541 (1976)
Non-patent Document 2: N. V. Chuyen, T. Kurata, H. Kato, J. Antibact. Antifung. Agents, 11, 69-75 (1983)
Non-patent Document 3: N. V. Chuyen, H. Kato, Agric. Biol. Chem., 46, 2795-2801 (1982) 3-1128 (2004)

### DISCLOSURE OF THE INVENTION

### PROBLEMS TO BE SOLVED BY THE INVENTION

An object of the present invention is to provide an antiviral agent.
Another object of the present invention is to provide an antiviral agent with high antiviral activities and low side effects (cytotoxicity).

### MEANS FOR SOLVING THE PROBLEMS

The present invention provides an antiviral agent as follows:
(1) An antiviral agent comprising as an effective component at least one member selected from the group consisting of 5,7,4'-trihydroxy-3',5'-dimethoxyflavone, 3-hydroxypyridine, p-hydroxybenzaldehyde and vanillin.
(2) An antiviral agent comprising as an effective component 5,7,4'-trihydroxy-3',5'-dimethoxyflavone or 3-hydroxypyridine.
(3) An antiviral agent comprising as an effective component 5,7,4'-trihydroxy-3',5'-dimethoxyflavone.
(4) The antiviral agent according to any one of the above described (1) to (3), wherein the virus is a herpesvirus.
(5) The antiviral agent according to any one of the above described (1) to (3), wherein the virus is a cytomegalovirus.
(6) The antiviral agent according to any one of the above described (1) to (3), wherein the virus is a human cytomegalovirus.
(7) The antiviral agent according to the above described (3), wherein the virus is an influenza virus.
(8) The antiviral agent according to the above described (7), wherein the virus is an influenza virus A or influenza virus B.
(9) The antiviral agent according to the above described (7), wherein the virus is an avian influenza virus.

The term "virus" used in the present invention includes animal virus, insect virus, plant virus, bacterial virus (bacteriophage), in particular, the term means virus that has an undesirable effect on mammalians including human as well as animals and plants including birds.
Examples of the virus against which antiviral agent according to the present invention is particularly effective include herpesvirus and influenza virus, in particular, cytomegalovirus, cytomegalovirus such as in particular human cytomegalovirus, simian cytomegalovirus, mouse cytomegalovirus and human influenza virus and avian influenza virus, such as influenza virus A, influenza virus B, and influenza virus C.

### EFFECTS OF THE INVENTION

The antiviral agent according to the present invention has high antiviral activities against various viruses, in particular herpesvirus, especially cytomegalovirus particularly human cytomegalovirus; influenza virus, in particular human influenza virus A, influenza virus B, influenza virus C and avian influenza virus.
The antiviral agent according to the present invention has superior advantages in that it has low cytotoxicity.

### EMBODIMENTS FOR CARRYING OUT THE INVENTION

The present inventors have clarified the nature of the antimicrobial effect of Kumazasa (Sasa albo marginata) and in order to specify effective antimicrobial components, they fractionated a hot water extract of Kumazasa leaf by using various solvents, separated and purified by using various separation and purification means, and investigated its antiviral activities for each component.
As a result, they discovered that 5,7,4'-trihydroxy-3',5'-dimethoxyflavone (hereinafter also referred to as "tricin"), 3-hydroxypyridine, p-hydroxybenzaldehyde, and vanillin, in particular 5,7,4'-trihydroxy-3',5'-dimethoxyflavone and 3-hydroxypyridine, especially 5,7,4'-trihydroxy-3',5'-dimethoxyflavone (tricin) exhibited superior antiviral activities against various viruses, thereby completing the present invention.

The present invention will now be described concretely.
The effective component according to the present invention, 5,7,4'-trihydroxy-3',5'-dimethoxyflavone, 3-hydroxypyridine, p-hydroxybenzaldehyde and vanillin are extracted from Kumazasa. Yet, needless to say, synthetic component and/or one obtained from other sources may be also used. For instance, 5,7,4'-trihydroxy-3',5'-dimethoxyflavone (tricin) existed abundantly in and can be extracted from plants other than Kumazasa, in particular many plants belonging to Gramineae, for example, rice plant (rice), wheat, maize, barley, rye, sugarcane, bamboo (Take), Japanese pampas grass, pampas grass, leaves and stems of Japanese nutmegs such as reed (also called phragmites or Yoshi) and so on. Concrete plant names are as follows:
Rice plant, wheat, barley, Avena fatua, rye, proso millet, foxtail millet, Japanese barnyard millet, maize, finger millet, sorghum, Take, wild rice (Makomo), sugarcane, job's tears, reed, Japanese pampas grass, Sasa, Arundo donax, Cortaderia argentea and lawn grass.

Tricin may be separated and purified by subjecting leaf, stem or the like of the above-described plants to extraction with suitable solvent, and by using separation and purification means such as HPLC. For example, tricin may be purified by concentrating a water extract, extracting the solid residue with alcohol or aqueous alcohol (for example. methanol, ethanol, aqueous methanol or aqueous ethanol), dissolving the solid content in water, and by partitioning the resulting solution with ethyl acetate.

The antiviral agent according to the present invention which comprises as an effective ingredient at least one selected from the above-described compounds (vanillin, p-hydroxybenzaldehyde, 3-hydroxypyridine, 5,7,4'-trihydroxy-3',5'-dimethoxyflavone) (herein also referred to as "compound of the invention") may be used in various forms. For example, the antiviral agent according to the present invention is suitable for a mucosal-protective composition, prophylactic and/or therapeutic composition for virus infection, preservative, and an antiviral agent for a filtration apparatus or the like.
The formulation of the antiviral agent according to the present invention may be any form of liquid, solid, gas, gel, or aerosol. The antiviral agent according to the present invention may be administered either orally or parenterally.
Examples of the oral administration mode include tablets, balls, powders, liquids, and food forms such as chewing gums, candies, chocolates, bread, cookies, buckwheat noodles, Japanese wheat noodles, foods such as various drinkable preparations, drinking water, and condiments. Examples of the parenteral administration mode include injection solutions, formulations for topical administration (such as creams and ointments), suppositories and vaginal administration (such as tampons) and the like. Examples of the formulation for topical administration include carriers such as gauzes made of natural or synthetic fibers in which carriers the antiviral agent of the present invention is impregnated, and cosmetics such as lipsticks and the other form of cosmetics (lotions, oils, soap, face lotions, facial creams and the like) and bath salts in which the antiviral agent according to the present invention is incorporated, the form of semi-solid or liquid such as essence, shampoo, body soap and cleansing foams. The form of eye drops, collutories for gargling or the like may be used. The form of wound nebulae for treatment of wound, nebulae for treatment of pharyngeal inflammation and the like may be also used.
The antiviral agent according to the present invention may be useful as an antiviral agent for not only human but also mammals other than human, birds, fishes, crustacea, insects, amphibia, and reptiles. Hence, the antiviral agent according to the present invention may be used as an antiviral agent for these animals (for example, a pharmaceutical for pets, animal feeds, pet food or the like). Moreover, the antiviral agent according to the present invention is useful as an antiviral agent for various plants as well as animals, and also useful as a preservative.

For the production of the antiviral agent of the present invention in various formulations, base materials such as oily components used in usual pharmaceutical compositions, cosmetics, compositions for skin and the like, moisturizers, preservatives and the like, in addition to a prescribed amount of the above-described compound, may be used.
Water used in the antiviral agent is not restricted, and it may be tap water, natural water, purified water or the like. In general, water with a high purity such as ion exchanged water is preferably used.
Examples of the oily component include animal oils such as squalane, beef tallow, lard, horse oil, lanolin, beeswax and the like; plant oils such as olive oil, grape seed oil, palm oil, jojoba oil, germ oil (e.g. rice germ oil), sesame oil, canola oil, safflower oil, salad oil and the like; synthetic oils such as liquid paraffin, higher fatty acid ester (for example, octyl palmitate, isopropyl palmitate, octyldodecyl myristate), silicone oil and the like; and semi synthetic oils.
Two or more of the oily components may be used appropriately in combination depending on what is desired, for example, protection of skin, emollient effect (to cover the surface of the skin with thin layer to protect skin from drying, and to give the skin tenderness and elasticity), light texture and so on. One of the preferred examples is a combination of squalane, olive oil and octyldodecyl myristate.
To adjust hardness and/or fluidity of the antiviral agent, solid fats such as stearic acid, stearyl alcohol, behenic acid, cetanol, vaseline and the like may be used. Preferably stearic acid and cetanol are used in combination.

In order to produce the antiviral agent of the present invention as a cream composition, a creaming agent which makes a mixture of the compound of the invention, water and oily component creamy is used. The creaming agent is not restricted, and in general a combination of glyceryl monostearate and self-emulsifying glyceryl monostearate (a mixture of glyceryl monostearate and emulsifier) is used.
The antiviral agent according to the present invention may be used in conjunction with other antiviral agents.
The antiviral agent according to the present invention may be comprise (a) stabilizer(s), moisturizer(s), wound healing agent(s), preservative(s), surfactant(s) or the like as required.
Examples of the stabilizer include a combination of carboxyvinyl polymer and potassium hydroxide, polyethylene glycol distearate and the like. Especially, polyethylene glycol sesquistearate (a 1:1 mixture of polyethylene glycol distearate and polyethylene glycol monostearate) (a molecular weight of polyethylene glycol is 1,000 to 20,000) is preferred, because it has a high stability and the composition comprising it does not separate into water and oil, and moreover it can adjust effectively the hardness of the cream composition which is applied to skin.
Examples of the moisturizer include sodium hyaluronate, collagen, aloe extract (especially preferred is an aloe extract (2) originated from Aloe arborescens var. natalensi), urea, 1,3-butylene glycol, glycerin, trehalose, sorbitol, amino acid, sodium pyrrolidone carboxylate and the like.
Examples of the wound healing agent include allantoin, dipotassium glycyrrhizinate, licorice extract, Artemisia extract and the like.

The preservative is supplementarily used. Examples of the preservative include sodium benzoate, lower alkyl esters of para-hydroxybenzoic acid (e.g. esters called paraben such as methyl, ethyl, propyl or butyl ester), sodium propionate, mixed fatty acid ester (a mixture of glyceryl caprate, polyglyceryl-2 laurate and polyglyceryl-10 laurate), phenoxyethanol, photosensitive pigment No. 201 (yellow pigment), 1,2-pentanediol and the like, and preferred are paraben, mixed fatty acid ester and 1,2-pentanediol.
Examples of the surfactant include sodium N-acyl-L-glutamate, polyoxyethylene sorbitan monostearate and the like.
Further as required, perfume components such as orange oil, lemon oil, bitter orange oil, flavoring agents and the like may be contained.

A mucosal-protective composition, prophylactic and/or therapeutic composition for viral infection which comprises the antiviral agent according to the present invention are the agents which effectively suppress invasion of infectious viruses into a body through a mucosa of eye, nose, throat, ear, anus, genital organs or the like, and through wounds and skin, thereby preventing and/or treating viral infection including nosocomial infection. More concrete examples of the prophylactic and/or therapeutic composition for viral infection include mucosal-protective cloths and compositions for oral cavity application.

A mucosal-protective cloth is an air permeable carrier in which the compound according to the present invention is incorporated by a method such as impregnation, spraying or the like, wherein the air permeable carrier is, for example, natural fibers such as silk, cotton, hemp and the like, synthetic fibers such as polyurethane, polyethylene, polypropylene, nylon, polyester, acryl and the like, semi-synthetic fibers, or mixed fibers of two or more of these fibers; or yarns, woven fabric, knit, nonwoven fabric or paper thereof.
The term "protective cloth" herein includes not only cloths but also fibers per se, yarns, papers and the like for convenience.
Concrete examples of the form of the mucosal-protective cloth include not only protective cloths directly contacting with mucosa or skin which are classified as sanitary goods such as gauzes, masks, eye patches, sanitary belts, sanitary napkins, bandages, toilet papers, gauzes for hemorrhoids treatment, earplugs, liquid bandages and the like, but also articles contacting directly or indirectly with skin, e.g., clothes such as white robes and the like; small clothing articles such as gloves, hats, socks, Japanese socks and the like; beddings such as bed sheets, quilt covers, pillowcases, articles for bedding and the like; interior accessories and interior finishing materials such as curtains, wallpapers, carpets and the like; medical materials such as surgical sutures and the like, and so on.

Examples of the composition for oral cavity application comprising the antiviral agent according to the present invention include gummies, jellies, troches, candies, chewing gums, chocolates, tablets, balls, mouth washes, collutories for gargling, tooth pastes, films for application to mucosa, nebulae for treatment of pharyngeal inflammation and the like.

The antiviral agent according to the present invention has high antiviral activities and the solid concentration of even about 1000 ppm to 1 ppm exhibits sufficient antiviral activities in terms of the compound according to the present invention.
The antiviral agent according to the present invention may be administrated for human adult at a dose of 1 to 5000 mg, preferably 5 to 2000 mg, further preferably 10 to 1000 mg once a day or dividedly in twice to six times a day in the view of symptoms, age, weight and the like.
When the compound according to the present invention is incorporated in compositions for oral cavity application such as gummies, jelly, troches, candies, chewing gums, tablets, balls (for example sasatan), mouth washes, collutories for gargling, tooth pastes, films for application to mucosa and the like, the compound of the invention may be added to the source material of the composition for oral cavity application in any one of the steps for producing the composition for oral cavity application. For instance, the compound of the invention is appropriately added in an amount of 2 to 20× (1/10 to 1/100,000) % by mass, more preferably about 6 to 15× (1/10 to 1/100,000) % by mass, most preferably about 8 to 12× (1/10 to 1/100,000) % by mass in terms of solid content.

Examples of the formulations of the antiviral agent according to the present invention include capsules, dry syrups, tablets, balls, powders, liquids, pharmaceutical preparation for nasal drops which directly applied to nasal cavity and/or pharynx such as for example solutions and gels for nasal drops and aerosol such as oral cavity spray, and pharmaceutical preparation for percutaneous absorption such as ointments, emulsions and creams. These can be prepared by a conventional method.
Examples of the base component for preparing the above described formulation of the antiviral agent include vehicles such as glucose, lactose, sucrose, starch syrup, dextrin, cyclodextrin, starch and the like; binders such as gum arabic, sodium carboxymethyl cellulose, crystalline cellulose, gum base and the like; disintegrators such as starch and the like; lubricants such as magnesium stearate, sucrose fatty acid esters and the like; refrigerants such as perfumes, chlorophyll, peppermint, 1-menthol and the like; higher alcohol such as octyl dodecanol and the like; fatty acid esters such as myristic acid isopropyl, adipic acid diisopropyl, isopropyl palmitate and the like; suspending agents such as polysorbate 80, polyoxyethylene hydrogenated castor oil and the like; high molecular compounds such as carboxyvinyl polymer, hydroxypropylcellulose, polyvinylpyrrolidone, sodium hyaluronate and the like; polyalcohol such as glycerin, propylene glycol 1,3-butylene glycol and the like; pH regulators such as diisopropanolamine, sodium hydroxide, monobasic potassium phosphate, dibasic sodium phosphate and the like; stabilizing agents such as dibasic sodium phosphate, sodium chloride, sodium thiosulfate, sodium sulfite, edetate sodium and the like; and preservatives such as methylparaben, propylparaben, benzalkonium chloride, benzethonium chloride and the like.

In cases where the antiviral agent according to the present invention is used in the form of a protective cloth, the compound according to the present invention may be incorporated in a part of a protective cloth (e.g. gauze) with which part mucosa or wound contacts, and the protective cloth may be changed to a fresh one once to three times a day. When the mucosal-protective composition is used in the form of surgical sutures, invasion of viruses through the sutured area can be effectively suppressed, thereby promoting recovery of the operative site.
When the antiviral agent according to the present invention is used in the form of a protective cloth in order to prevent nosocomial infection in a hospital or the like, the effect of the antiviral agent according to the present invention to prevent infection continues for a long time. If the effect is reduced by washing, the protective cloth may be appropriately changed, or the process for incorporating the compound according to the present invention in the protective cloth again may be performed.

In cases where the antiviral agent according to the present invention is used in the form of a composition for oral cavity application, viral infections can be prevented and the growth of the virus can be suppressed very simply by appropriately placing into the mouth as required. The composition in sustained release form such as chewing gums, candies or the like can exert its effect for a long time and therefore such form is advantageous.
The antiviral agent according to the present invention may be used in the form of lotions or oils. By applying to skin a lotion or oil comprising the compound according to the present invention in an amount of 2 to 20× (1/10 to 1/100,000) % by mass in terms of the solid content, infections of infectious bacteria can be prevented and their growth can be suppressed very simply.

### Filtration Apparatus

The present invention also provides an air filtration apparatus comprising as an effective component the compound of the invention. Examples of the concrete form of the apparatus include filters which are applied to a region through which the air passes, for example, filters used in ventilation fans, air-conditioners, car air-conditioners, air admission ports, air exhaust ports, screen doors, air cleaners and the like. The material constituting the filter is not restricted and examples of the material include woven fabrics, knits, nonwoven fabrics, papers and the like, which are made of natural fibers such as silk, cotton, wool, hemp and the like, synthetic fibers such as polyurethane, polyethylene, polypropylene, nylon, polyester, acryl and the like, semi-synthetic fibers, or mixed fibers of two or more of these fibers. The compound according to the present invention may be incorporated in such a filter by a method such as impregnation, spraying or the like, and the filter may be dried. The amount of the compound according to the present invention comprised in the filter is preferably 2 to 20× (1/10 to 1/100,000) % by mass, more preferably about 6 to 15× (1/10 to 1/100,000) % by mass, most preferably about 8 to 12× % by mass in terms of solid content.

### Example 1 (An antiviral action of Kumazasa extract against herpesvirus)

An antiviral action of Kumazasa extract against herpesvirus was examined by the method below.

### Method:

Human fetal lung fibroblast (MRC-5) cells cultured to monolayer in a Petri dish were infected with human cytomegalovirus (HCMV, Towne strain (standard strain) and 93-1R strain (ganciclovir resistant strain)). Medium containing a diluting aqueous solution of a commercially available Kumazasa extract (TWEBS sold by Hououdou Co., Ltd.(solid concentration 50% by mass )), varied concentrations of the component separated and purified from Kumazasa extract, or a commercially available antiviral agent was added and cell culture was started in 5% CO₂ incubator.
(1) On Days 1, 3, and 6 from the culturing, presence of cytopathic effect (CPE) and cell conditions were examined under microscope and effects of each sample on CPE were evaluated.
(2) On Day 6 from the culturing, the number of virus particles produced in the culture medium was measured by plaque technique and effects of each sample on virus production were evaluated.
(3) On Days 1, 3, and 6 after the culturing, virus-infected cells in each group were solubilized in a buffer for electrophoresis, proteins were separated by electrophoresis, followed by Western blotting to comparatively evaluate expression of viral proteins. In particular, with regard to expression of the immediate early (IE) protein, which is a significantly important protein expressed in the early phase of several hours after infection and functioning as a trigger protein of subsequent viral replication, and the late (Late) protein such as DNA synthetase, effects of each sample were comparatively evaluated.

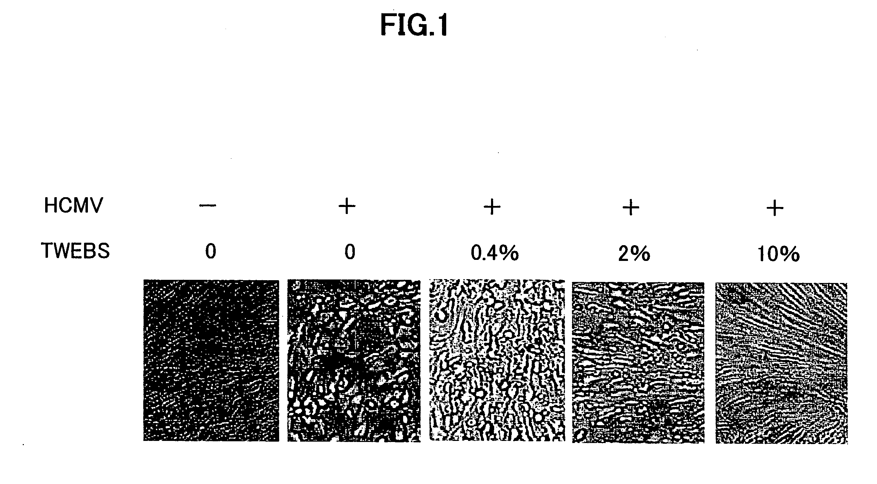

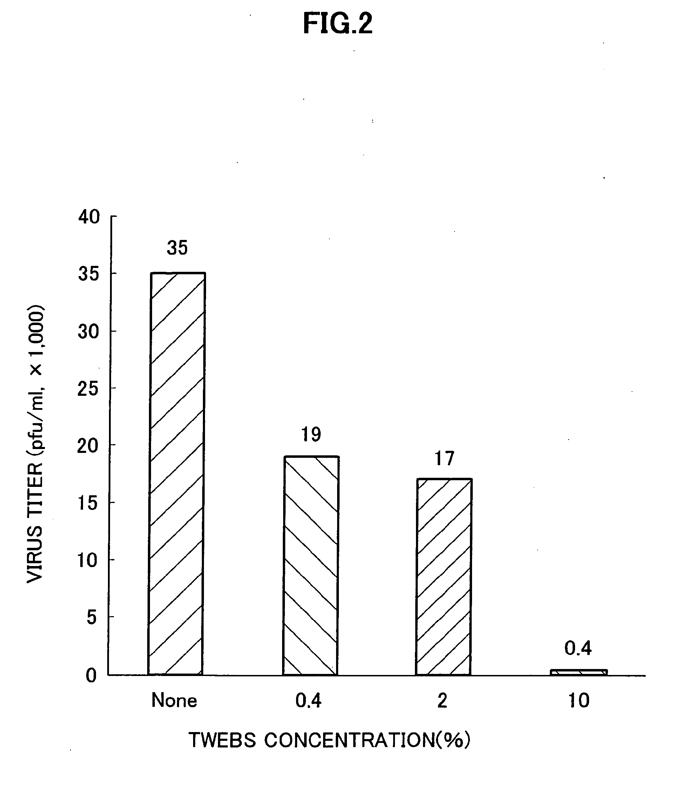

The cytopathic effect (CPE) and suppressive effect on viral proliferation when the diluting aqueous solution of Kumazasa extract (TWEBS sold by Hououdou Co., Ltd. (solid concentration 50% by mass)) was used are respectively shown in Figure 1 and Figure 2. In both Towne and 93-1R strains, the cytopathic effect (CPE) and suppressive effect on viral proliferation were observed at least at a concentration of 0.4%. This indicates that the components in Kumazasa extract (in particular tricin) were to be effective against not only normal cytomegalovirus but also ganciclovir resistant virus and the discovery was made ahead of others.

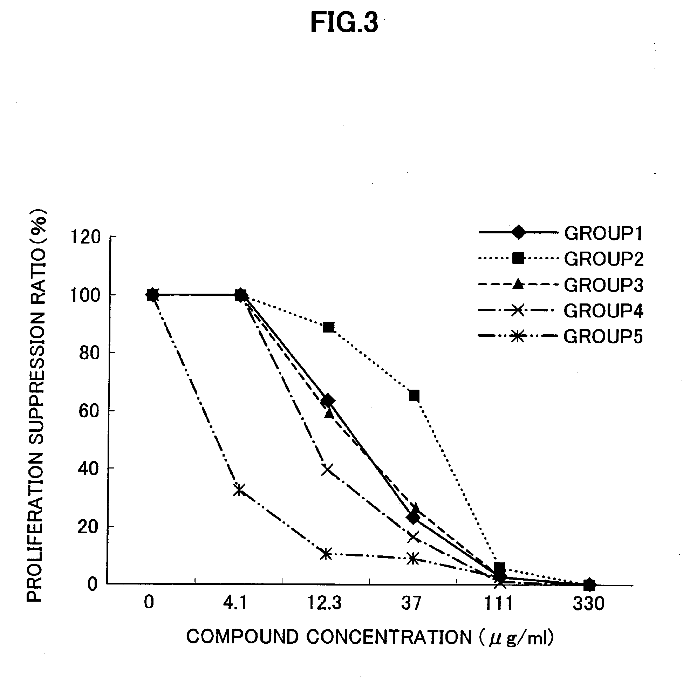

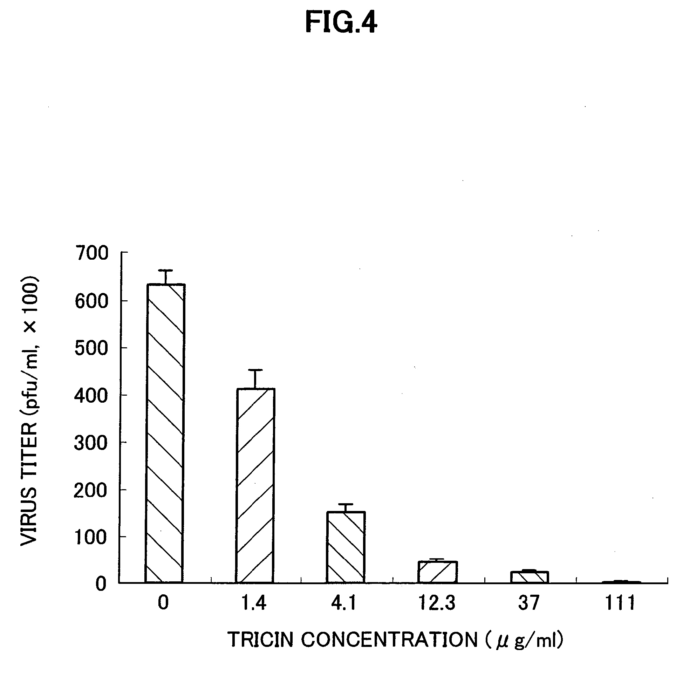

An antiviral action of the separated and purified components from Kumazasa extract against herpesvirus
As the separated and purified component from Kumazasa extract p-coumaric acid (group 1), vanillin (group 2), p-hydroxybenzaldehyde (group 3), 3-hydroxypyridine (group 4), 5,7,4'-trihydroxy-3',5'-dimethoxyflavone (tricin) (group 5) were used.
The suppressive effect of each compound on HCMV proliferation is shown in Figure 3.
Also, a relationship of a concentration of tricin and virus titer is shown in Figure 4. It can be seen that proliferation of the virus is virtually completely inhibited at a tricin concentration of 111 µg/ml. Further, IC₅₀ (50% inhibitory concentration), (cytotoxicity) (µg/ml), EC₅₀ (50% effective concentration) (µg/ml) and SI (selection index) of each type of compound are shown in Table 1.

**[Table 1]**

| Compound | IC₅₀ | EC₅₀ | SI |
|---|---|---|---|
| p-coumaric acid | 187 | 8.2 | 22.8 |
| Vanillin | 215 | 48.0 | 4.5 |
| p-hydroxybenzaldehyde | 305 | 12.8 | 23.8 |
| 3-hydroxy pyridine | 263 | 3.4 | 77.4 |
| Tricin | 2050 | 1.7 | 1205.8 |
| Ganciclovir | | 0.3-0.8 | |

From Table 1, it can be seen that the compound according to the present invention, in particular tricin (flavone derivative) has significantly low cytotoxicity and low effective concentration (high activity) and thus selection index (IC₅₀/EC₅₀) is markedly high.

### Example 2 (An antiviral action of tricin against influenza virus)

An antiviral action of tricin against influenza virus was investigated by the method below.
Cells: MDCK cells
Type of influenza virus:
A/Hiroshima/52/2005, H3N2
B/Malaysia/2506/2004
Each virus was proliferated in MDCK cells. After the virus titer was determined, the virus was frozen and stored in -80°C, and, as needed, thawed to be used.

### Method: A conventional plaque technique was followed.

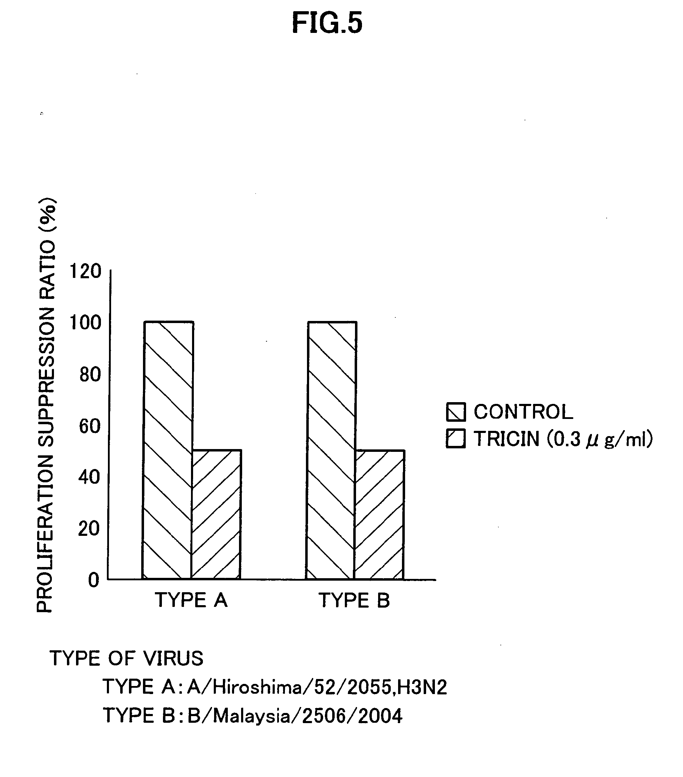

That is, each virus was absorbed by MDCK cells cultured to monolayer in a plastic Petri dish in 5% CO₂ incubator at room temperature for an hour. After unabsorbed virus was removed, 0.6% agar supplemented with trypsin and containing varied concentration of tricin was added and solidified at room temperature. And then the resultant was cultured in 5% carbon dioxide gas in an incubator for two days.
After the culture was terminated, the cells were fixed with 10% formalin and the agar was removed. The cells were then stained with 0.03% methylene blue solution and the number of plaques appeared was counted.
An antiviral action (proliferation suppression ratio = pt/pc × 100%) was calculated by dividing the number of the plaques in the test group added tricin (pt) by the number of the plaques in the control group added no tricin (pc). At a tricin concentration of 0.3 µg/ml, the proliferation suppression ratio for influenza type A virus was 52% and the proliferation suppression ratio for influenza type B virus was 48%. The results are shown in Fig. 5.
The above described results satisfactorily demonstrate that tricin exhibits high actions to suppress proliferation of other influenza virus such as avian influenza virus and the like.

### Pharmaceutical preparation example1 (capsules)

Tricin (250mg) was placed in a capsule to prepare the antiviral agent according to the present invention.

### Pharmaceutical preparation example 2 (tablets)

Tricin and lactose were mixed and molded to prepare a tablet containing 100 mg of tricin per tablet.

### BRIEF DESCRIPTION OF THE DRAWINGS

Figure 1 is a photograph under microscope showing suppression of the cytopathic effect of Kumazasa extract (TWEBS) after viral infection.
Figure 2 is a graph showing effect of Kumazasa extract (TWEBS) to suppress viral proliferation.
Figure 3 is a graph showing effect of the isolated components in Kumazasa extract (TWEBS) to suppress viral proliferation.
Figure 4 is a graph showing effect of tricin (flavone derivative) to suppress proliferation of human cytomegalovirus.
Figure 5 is a graph showing effect of tricin to suppress proliferation of influenza virus.

## Claims

1. An antiviral agent comprising as an effective component at least one member selected from the group consisting of 5,7,4'-trihydroxy-3',5'-dimethoxyflavone, 3-hydroxypyridine, p-hydroxybenzaldehyde and vanillin.

2. An antiviral agent comprising as an effective component 5,7,4'-trihydroxy-3',5'-dimethoxyflavone or 3-hydroxypyridine.

3. An antiviral agent comprising as an effective component 5,7,4'-trihydroxy-3',5'-dimethoxyflavone.

4. The antiviral agent according to any one of claims 1 to 3, wherein the virus is a herpesvirus.

5. The antiviral agent according to any one of claims 1 to 3, wherein the virus is a cytomegalovirus.

6. The antiviral agent according to any one of claims 1 to 3, wherein the virus is a human cytomegalovirus.

7. The antiviral agent according to claim 3, wherein the virus is an influenza virus.

8. The antiviral agent according to claim 7, wherein said virus is an influenza virus A or influenza virus B.

9. The antiviral agent according to claim 7, wherein said virus is an avian influenza virus.
